# EUROPEAN PATENT SPECIFICATION

(11) **EP 1 705 245 B1**
(45) Date of publication and mention of the grant of the patent: **20.05.2009**
(21) Application number: 05102356.2
(22) Date of filing: 23.03.2005
(51) Int. Cl.: C12N 5/06, C12N 5/08, A61P 25/00

(54) **Neural stem cells**
Neurale Stammzellen
Cellules souches neurales

(43) Date of publication of application: 27.09.2006
(73) Proprietor: Stiftung CAESAR, 53175 Bonn (DE)
(72) Inventor: Thie, Michael, Dr., 53175 Bonn (DE); Degistirici, Özer, Dr., 53177 Bonn (DE)
(74) Representative: Remus, Alvaro Johannes

(56) References cited:
- WO-A-03/066840
- US-A- 5 885 829
- MIURA MASAKO ET AL: "SHED: stem cells from human exfoliated deciduous teeth" PROCEEDINGS OF THE NATIONAL ACADEMY OF SCIENCES OF USA, NATIONAL ACADEMY OF SCIENCE. WASHINGTON, US, vol. 100, no. 10, 13 May 2003 (2003-05-13), pages 5807-5812, XP002279268 ISSN: 0027-8424
- GRONTHOS S ET AL: "STEM CELL PROPERTIES OF HUMAN DENTAL PULP STEM CELLS" JOURNAL OF DENTAL RESEARCH, INTERNATIONAL ASSOCIATION FOR DENTAL RESEARCH, US, vol. 81, no. 8, August 2002 (2002-08), pages 531-535, XP009008972 ISSN: 0022-0345
- MORSCZECK C ET AL: "Isolation of precursor cells (PCs) from human dental follicle of wisdom teeth" MATRIX BIOLOGY, ELSEVIER, vol. 24, no. 2, April 2005 (2005-04), pages 155-165, XP004884874 ISSN: 0945-053X & MORSCZECK C ET AL: "Isolation of precursor cells (PCs) from human dental follicle of wisdom teeth" MATRIX BIOLOGY, [Online] 12 February 2005 (2005-02-12), Retrieved from the Internet: URL:http://www.sciencedirect.com/science?_ ob=ArticleURL&_udi=B6VPM-4FG2X31-1&_coverD ate=04%2F30%2F2005&_alid=309454955&_rdoc=1 &_fmt=&_orig=search&_qd=1&_cdi=6210&_sort= d&view=c&_acct=C000049880&_version=1&_urlV ersion=0&_userid=987766&md5=a27aeacf7a2ebf a5c0c7e5ea029fb2b0> [retrieved on 2005-09-02]
- SEO B M ET AL: "Investigation of multipotent postnatal stem cells from human periodontal ligament" LANCET THE, LANCET LIMITED. LONDON, GB, vol. 364, no. 9429, 10 July 2004 (2004-07-10), pages 149-155, XP004746088 ISSN: 0140-6736
- WISE G E ET AL: "Cellular, molecular, and genetic determinants of tooth eruption." CRITICAL REVIEWS IN ORAL BIOLOGY AND MEDICINE : AN OFFICIAL PUBLICATION OF THE AMERICAN ASSOCIATION OF ORAL BIOLOGISTS. 2002, vol. 13, no. 4, 2002, pages 323-334, XP002343589 ISSN: 1045-4411

## Description

Subject of the invention is a method for generating matrix-independent neural stem cells (NSC) in vitro, wherein stem cells are obtained from tissue from the dental follicle of tooth or wisdom tooth and differentiated to the neural stem cells.

Neurodegenerative diseases are characterized by the loss of specific subsets of neurons, and whilst drug therapies exist for some of these disorders, none of them are curative. Neural tissue has a limited capacity for repair after injury, and adult neurogenesis is limited to select regions of the brain (Gage, 2000; Magavi, 2000; Rakic, 2000 and Temple and Alvarez-Buylla, 1999).

Neural cells can be generated from embryonic stem cells (ESCs) and neural stem cells (NSCs) from embryonic tissue (Bain et al., 1995; Bruestle et al., 1999; Lindvall et al., 1990; McKay, 1997) or from fetal tissues, the most successful being the transplantation of human fetal tissue into Parkinson's patients (Freed et al., 2001).

The use of ESCs and NSCs which are derived from embryonic and fetal tissues is limited by various ethical and logistical constraints, and thus tissues from adults may be an alternative source of stem cells and progenitor cells as shown in the case of bone marrow (Brazelton et al., 2000; Mezey et al., 2003; Sanchez-Ramos et al., 2002; Jiang et al., 2002) and from developing (Vescovi et al., 1999; Svendsen et al. 1997) or adult brain (Studer et al., 1998; Wu et al., 2002; Daadi and Weiss, 1999; Vicario-Abejon et al., 2000). Further sources already available include bone marrow stromal cells (Prockop et al., 2000; Hofstetter et al., 2002), stem cells from dermis (Toma et al., 2001) and neural crest stem cells from the gut and sciatic nerve (Bixby et al., 2002; Kruger et al., 2002).

It has recently been shown that exfoliated human deciduous teeth and dental pulp contains a population of multipotent stem cells with the capacity to differentiation into several different cell lineages, including glial and nerve cells (Miura et al., 2003; Gronthos et al., 2002).

In WO 03/066840 it is disclosed that adherent growing pluripotent embryonic-like stem cells have been isolated derived dental follicle from teeth. The inventors found that the pluripotent stem cells have the potential for extended renewal of teeth, periodontium and related tissues such as bones. The cells are cultured as biological membranes or scaffolds, which are necessary to support differentiation into mesothelic/endothelic cells, into blood vessels, into ectodermal tissue or into neural tissue of teeth (periodontium).

So far, the work with human embryonic stem cells in cell replacement therapies has been mostly hampered by the low cell numbers isolated from human fetal tissues and further by ethical and technical problems to produce these cells (Björklund et al., 2002).

The problem underlying the invention is thus to provide an alternative method for obtaining neural stem cells. The stem cells should be easily available without ethical or technical constraints, be obtainable in a simple process in high yield, and be fully capable of differentiation to neuronal cells.

Surprisingly, the problem of the invention is solved by a method wherein cells are isolated from tissue of the dental follicle of tooth or wisdom tooth. The isolated cells are cultured in serum-containing medium and sphere-forming cells are isolated therefrom. The spheres are cultured in a serum-free medium comprising bFGF and EGF and the primary spheres are mechanically dissociated. Spheroids are generated by culturing the sphere-forming cells again in a serum-free medium comprising bFGF and EGF. The spheroids are cultured in a serum-containing or serum-free medium comprising bFGF and EGF, wherein undifferentiated neural stem cells are isolated from a cell monolayer in serum-containing medium or from spheroids in serum-free medium formed from the dental follicle derived cells by neurogenic stimulation and acquire clear neuronal morphology and protein expression profile *in vitro.* This indicates the presence of a cell population in the dental follicle of the third molar with neuronal differentiation capacity that might provide benefits when implanted into central and peripheral nerve system..

According to the present invention, it is possible to directly generate neural stem cells from dental follicle without "membrane formation". "Free cells" in the context of the invention means that the NSC for isolation and cultivation are not embedded in a matrix, like a membrane or tissue. It is not necessary to use such a differentiation process to produce the neural stem cells. The invention is not related to the generation of periodontal neuronal cells as disclosed in WO 03/066840. The free stem cells of the invention may be single NSCs or homogenous cell aggregates like spheroids and neurospheres.

The procedure of the invention is suitable to generate a population of neural stem cells (NSCs), which are ectomesenchymal-derived dental follicle cells. According to the invention, cell aggregates (speroids, neurospheres) are cells are isolated from tissue of the dental follicle of tooth or wisdom tooth. The isolated cells are cultured in serum-containing medium and sphere-forming cells are isolated therefrom. The spheres are cultured in a serum-free medium comprising bFGF and EGF and the primary spheres are mechanically dissociated. Spheroids are generated by culturing the sphere-forming cells again in a serum-free medium comprising bFGF and EGF. The spheroids are cultured in a serum-containing or serum-free medium comprising bFGF and EGF, wherein undifferentiated neural stem cells are isolated from a cell monolayer in serum-containing medium or from spheroids in serum-free medium formed from the dental follicle derived cells by neurogenic stimulation and acquire clear neuronal morphology and protein expression profile *in vitro.* This indicates the presence of a cell population in the dental follicle of the third molar with neuronal differentiation capacity that might provide benefits when implanted into central and peripheral nerve system.

The invention comprises the isolation and expansion of multi-potent stem cells from the ectomesenchymal soft tissue of the third molar (wisdom tooth), which forms spheroids and progenitor cells with neuronal differentiation capacity.

The invention provides a method for obtaining neural stem cells, wherein stem cells are obtained from tissue from the dental follicle of tooth or wisdom tooth and differentiated to the neural stem cells. Such stem cells from tissue of the dental follicle are known from WO 03/066840. They can be isolated by the methods disclosed in WO 03/066840, especially the example on pages 20/21.

However, WO 03/066840 does not disclose the generation of neural stem cells. It only teaches that the dental follicle stem cells could be used for the creation of periodontal cell lines. In contrary, the free NSCs obtainable according to the invention are not limited to applications associated with the periodontium. This finding is very surprising, because in adult tissue like wisdom tooth there seems to be no need for stem cells capable of differentiation into cells which are not periodontal.

According to the invention, free neural stem cells are available which do not have to be generated in a tissue, biological membrane or scaffold. It is not necessary to add such tissues or membranes to promote differentiation.

Neural stem cells generated from wisdom teeth tissue are easily available and can be expanded in two strategies, i.e. from single spheres to a suspension culture of several spheres (i) or from single spheres to monolayer cultures and back to suspension cultures (ii). By this, a large number of cells can be generated to use for cell replacement strategies in treatment of neurodegenerative diseases. Furthermore, the neural induction medium used here allows growth and differentiation of neural cell types derived from spheroid-forming NSCs.

The availbility of NSCs derived from cells from the soft tissue of wisdom tooth (dental follicle) and the ability to differentiate into neurons, astrocytes or cholinergic neurons makes these cells ideal candidates for cell replacement therapies in neurodegenerative disorders, like Parkinson's disease or amyotrophic lateral sclerosis.

### Example:

Cells were enzymatically isolated from dissected soft tissue of wisdom teeth (dental follicle) by collagenase/Dispase treatment.

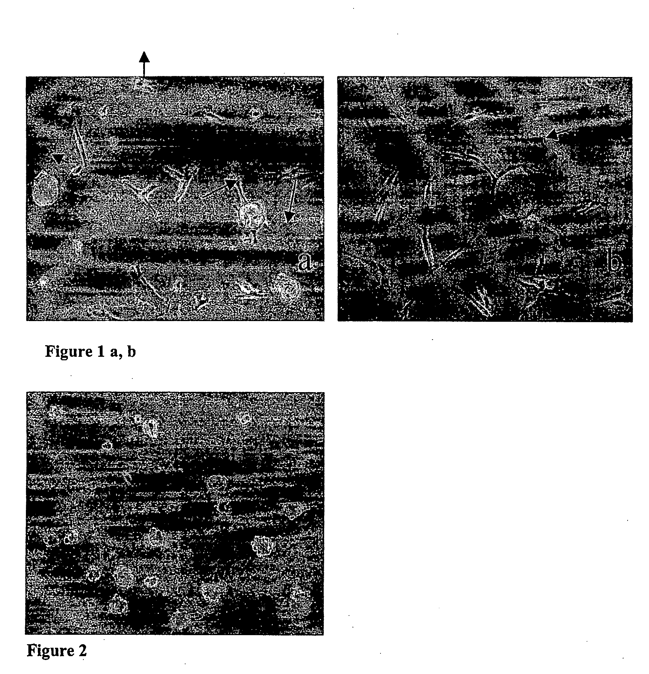

The ectomesenchymal cells were cultivated in FCS containing medium for 8-12 days. Some of cells adhered to the plastic culture flask while some died in suspension. However, some of the cells formed spheres (fig. 1).

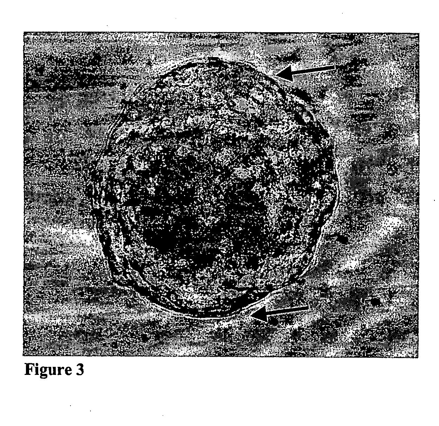

The floating spheres were transferred to new culture flasks after initial culturing in bFGF (40 ng/ml) and EGF (20 ng/ml), B27 (1:50) and neurobasal medium (invitrogen) containing medium or bFGF (50 ng/ml), EGF (25 ng/ml), ITS+Premix (1:50) and DMEM High glucose containing medium. Cells in spheres proliferated thereby forming large spheres which were successfully passaged and expanded (fig.2). The spheres seemed bright when viewed under a phase contrast microscope and showed cytoplasmic protrusions (cilia) at their surface (fig. 3)

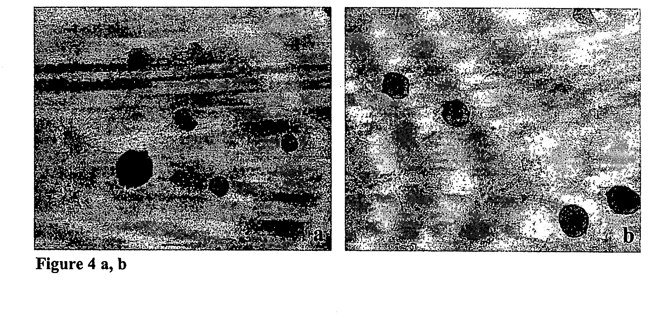

The primary spheres were mechanically dissociated into single cell suspensions and cultured again under sphere-forming conditions (ITS+Premix, bFGF, EGF, DMEM HG or bFGF, EGF, B27 and neurobasal medium; fig. 4a, b).

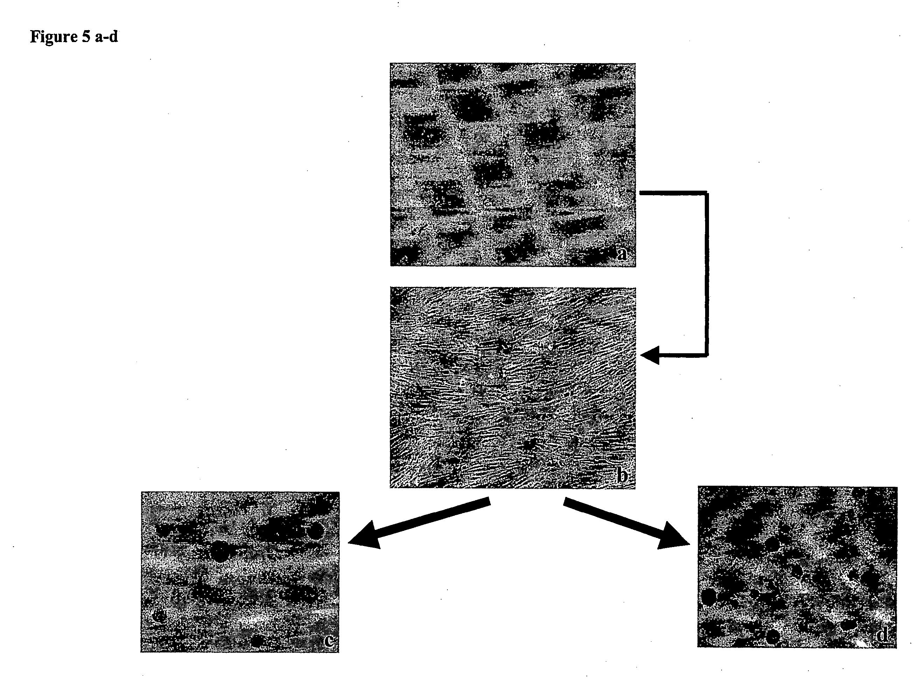

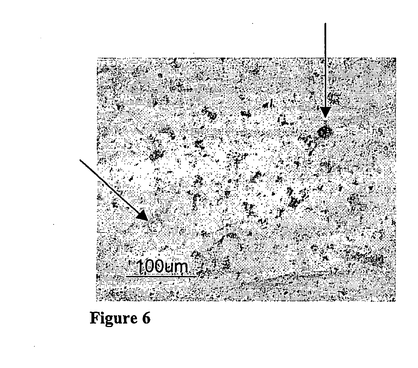

After each passage with both media the number of cells adhering to the plastic flask decreased while the number of cells forming spheroids increased (Fig. 5a). When the spheres grew in DMEM basic medium supplemented with serum, undifferentiated stem cells retained a flat polygonal fibroblast-like morphology (fig. 5b), in serum-free culture conditions cells formed again spheres (fig.5c, d). Immunocytochemistry with undifferentiated NSCs spheroids revealed that cells were stained for p75, a marker for neural growth factor receptor (fig. 6).

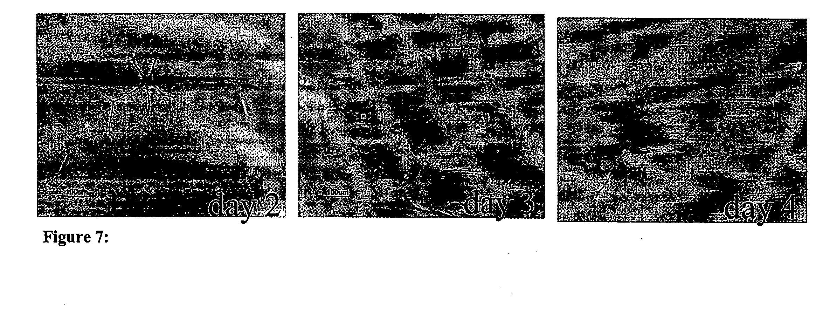

The potency of ex *vivo* expanded wisdom teeth-derived neural stem cells to generate neural cells was analyzed. The stem cells were seeded on fibronectin-coated glass cover slips. Cells were incubated in differentiation-medium containing DMEM, 15% heat inactivated FCS, penicillin/streptomycin/glutamine, 50 ng/ml β-NGF, 20 ng/ml FGF-b, 1 mM dibutyryl cAMP, 0.5 mM 3-isobutyl-1-methylxanthine, 10 µM all trans-retinoic acid. Differentiation-medium was changed every second day. Cells showed after each days neuronal morphology (fig. 7).

After one and two weeks, respectively, of differentiation-medium incubation, a subpopulation of neural stem cells stained for neurofilament, a marker of postmitotic neurons (fig. 8). Neural stem cells can be differentiated after one and two weeks into the glial lineage, expressing GFAP, a protein of the astrocytic cytoskeleton (fig. 9). A low percentage of NSCs showed staining for the inhibitory neurotransmitter GABA in basic growth medium after seven days of culture, indicating spontaneous differentiation (fig. 10b, c). At day 0, the cells were negative for GABA (fig. 10a). The staining for the inhibitory neurotransmitter GABA significantly increased by incubation in differentiation-medium (fig. 10d, e, f, g). After seven days of stimulation of NSCs with differentiation medium, immunoreactivity for choline acetyltransferase (CAT) was found indicating the development of a cholinergic neuronal subtype (fig. 11).

### Figures:

Figure 1: Initially formed spheres in primary culture a, b.
Figure 2: Spheres proliferated in neurobasal medium containing bFGF, EGF, B27.
Figure 3: Sphere with cilia in perimetry (400x).
Figure 4: a: Sphere formed in DMEM-high glucose containing ITS + premix, bFGF, EGF formed sphere; b: compared with neurobasal medium containing B27, EGF, bFGF-formed spheres.
Figure 5: Spheres; a: cultivated in neurobasal medium; b: mechanically dissociated and cultivated in FCS containing medium grown in monolayer; c: monolayer trypsinized and cultivated in neurobasal medium; d: in ITS + premix medium. The cells can be grown again in spheres.
Figure 6: Spheres formed initially in primary culture, positively stained with p75 antibody.
Figure 7:NSC treated with differentiation medium.

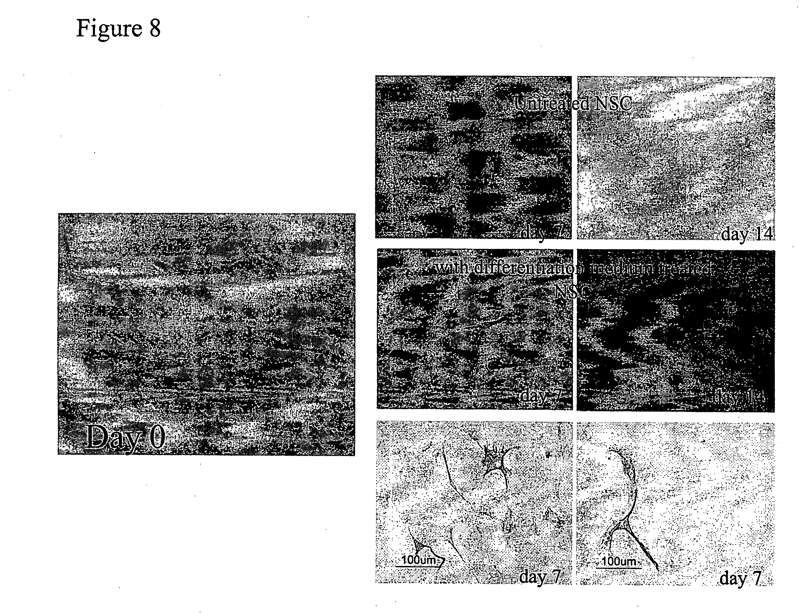

Figure 8: Neurofilament stained cells

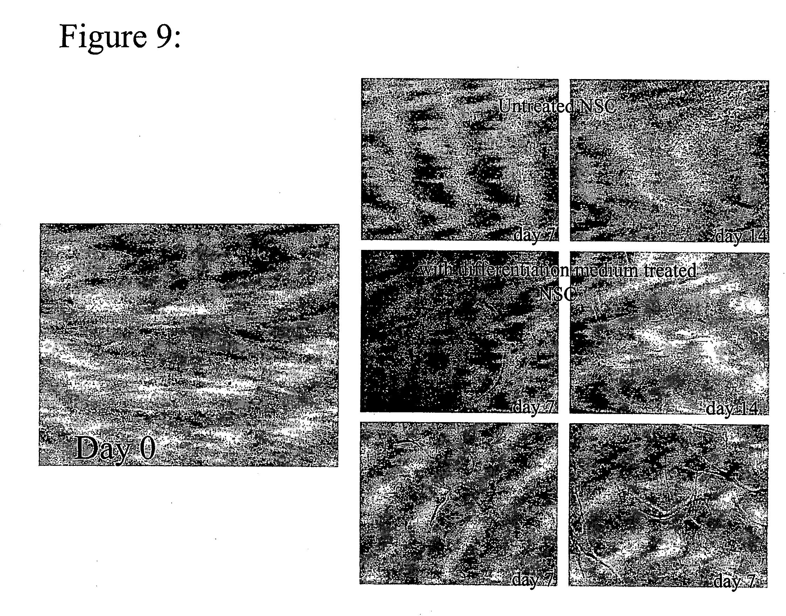

Figure 9: GFAP stained cells

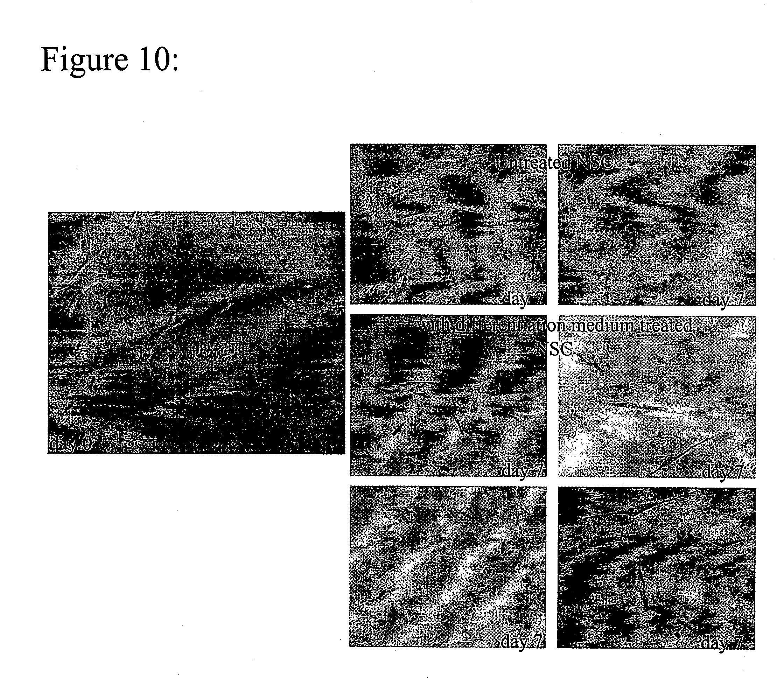

Figure 10: GABA stained cells

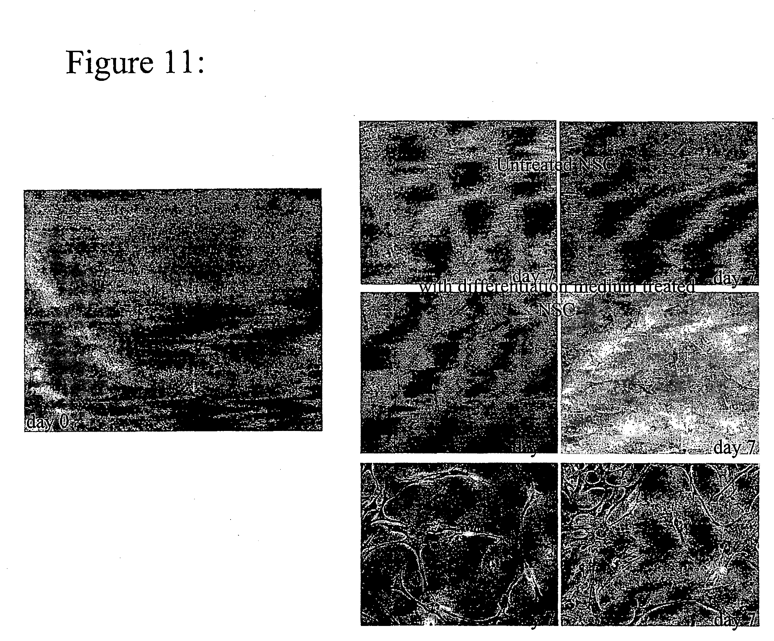

Figure 11: Choline acetyl transferase (CAT) stained cells

### Literature:

Gage FH. Mammalian neural stem cells. Science. 2000 Feb 25;287(5457):1433-8
Magavi SS, Leavitt BR, Macklis JD. Induction of neurogenesis in the neocortex of adult mice. Nature. 2000 Jun 22;405(6789):951-5.
Rakic S, Zecevic N. Programmed cell death in the developing human telencephalon. Eur J Neurosci. 2000 Aug; 12(8):2721-34.
Temple S, Alvarez-Buylla A. Stem cells in the adult mammalian central nervous system. Curr Opin Neurobiol. 1999 Feb;9(1):135-41.
Bain G, Kitchens D, Yao M, Huettner JE, Gottlieb DI.Embryonic stem cells express neuronal properties in vitro. Dev Biol. 1995 Apr;168(2):342-57.
Brustle O, Jones KN, Learish RD, Karram K, Choudhary K, Wiestler OD, Duncan ID, McKay RD. Embryonic stem cell-derived glial precursors: a source of myelinating transplants. Science. 1999 Jul 30;285(5428):754-6.
Lindvall O, Brundin P, Widner H, Rehncrona S, Gustavii B, Frackowiak R, Leenders KL, Sawle G, Rothwell JC, Marsden CD, et al. Grafts of fetal dopamine neurons survive and improve motor function in Parkinson's disease. Science. 1990 Feb 2;247(4942):574-7.
McKay R. Stem cells in the central nervous system. Science. 1997 Apr 4;276(5309):66-71.
Freed CR, Greene PE, Breeze RE, Tsai WY, DuMouchel W, Kao R, Dillon S, Winfield H, Culver S, Trojanowski JQ, Eidelberg D, Fahn S. Transplantation of embryonic dopamine neurons for severe Parkinson's disease. N Engl J Med. 2001 Mar 8;344(10):710-9.
Brazelton TR, Rossi FM, Keshet GI, Blau HM (2000). From marrow to brain: expression of neuronal phenotypes in adult mice. Science. Dec 1;290(5497):1775-9.
Mezey E, Chandross KJ, Harta G, Maki RA, McKercher SR (2000). Turning blood into brain: cells bearing neuronal antigens generated in vivo from bone marrow. Science. Dec 1;290(5497):1779-82.
Sanchez-Ramos JR.Neural cells derived from adult bone marrow and umbilical cord blood. J Neurosci Res. 2002 Sep 15;69(6):880-93.
Jiang Y, Jahagirdar BN, Reinhardt RL, Schwartz RE, Keene CD, Ortiz-Gonzalez XR, Reyes M, Lenvik T, Lund T, Blackstad M, Du J, Aldrich S, Lisberg A, Low WC, Largaespada DA, Verfaillie CM (2002). Pluripotency of mesenchymal stem cells derived from adult marrow. Nature. Jul 4;418(6893):41-9. Epub 2002 Jun 20.
Svendsen CN, Caldwell MA, Shen J, ter Borg MG, Rosser AE, Tyers P, Karmiol S, Dunnett SB. Long-term survival of human central nervous system progenitor cells transplanted into a rat model of Parkinson's disease. Exp Neurol. 1997 Nov;148(1):135-46.
Vescovi AL, Parati EA, Gritti A, Poulin P, Ferrario M, Wanke E, Frolichsthal-Schoeller P, Cova L, Arcellana-Panlilio M, Colombo A, Galli R.Isolation and cloning of multipotential stem cells from the embryonic human CNS and establishment of transplantable human neural stem cell lines by epigenetic stimulation. Exp Neurol. 1999 Mar;156(1):71-83.
Studer L, Tabar V, McKay RD. Transplantation of expanded mesencephalic precursors leads to recovery in parkinsonian rats. Nat Neurosci. 1998 Aug;1(4):290-5.
Wu P, Tarasenko YI, Gu Y, Huang LY, Coggeshall RE, Yu Y. Region-specific generation of cholinergic neurons from fetal human neural stem cells grafted in adult rat. Nat Neurosci. 2002 Dec;5(12): 1271-8.
Daadi MM, Weiss S. Generation of tyrosine hydroxylase-producing neurons from precursors of the embryonic and adult forebrain. J Neurosci. 1999 Jun 1;19(11):4484-97.
Vicario-Abejon C, Collin C, Tsoulfas P, McKay RD.Hippocampal stem cells differentiate into excitatory and inhibitory neurons. Eur J Neurosci. 2000 Feb;12(2):677-88.
Hofstetter CP, Schwarz EJ, Hess D, Widenfalk J, EI Manira A, Prockop DJ, Olson L.Marrow stromal cells form guiding strands in the injured spinal cord and promote recovery. Proc Natl Acad Sci U S A. 2002 Feb 19;99(4):2199-204.
Prockop DJ, Azizi SA, Colter D, Digirolamo C, Kopen G, Phinney DG.Potential use of stem cells from bone marrow to repair the extracellular matrix and the central nervous system. Biochem Soc Trans. 2000;28(4):341-5.
Toma JG, Akhavan M, Fernandes KJ, Barnabe-Heider F, Sadikot A, Kaplan DR, Miller FD. Isolation of multipotent adult stem cells from the dermis of mammalian skin. Nat Cell Biol. 2001 Sep;3(9):778-84.
Kruger GM, Mosher JT, Bixby S, Joseph N, Iwashita T, Morrison SJ.Neural crest stem cells persist in the adult gut but undergo changes in self-renewal, neuronal subtype potential, and factor responsiveness. Neuron. 2002 Aug 15;35(4):657-69.
Bixby S, Kruger GM, Mosher JT, Joseph NM, Morrison SJ. Cell-intrinsic differences between stem cells from different regions of the peripheral nervous system regulate the generation of neural diversity. Neuron. 2002 Aug 15;35(4):643-56.
Miura M, Gronthos S, Zhao M, Lu B, Fisher LW, Robey PG, Shi S.SHED: stem cells from human exfoliated deciduous teeth. Proc Natl Acad Sci U S A. 2003 May 13;100(10):5807-12. Epub 2003 Apr 25.
Gronthos S, Brahim J, Li W, Fisher LW, Cherma n N, Boyde A, DenBesten P, Robey PG, Shi S. Stem cell properties of human dental pulp stem cells. J Dent Res. 2002 Aug;81(8):531-5.
Schierholz J, Brenner N, Zeilhofer F, Hoffmann K. H, Morsczeck C. Pluripotent embryonic-like stem cells derived from teeth and uses thereof, 2003 PCT :WO 03/066840.
Bjorklund LM, Sanchez-Pernaute R, Chung S, Andersson T, Chen IY, McNaught KS, Brownell AL, Jenkins BG, Wahlestedt C, Kim KS, Isacson O. Embryonic stem cells develop into functional dopaminergic neurons after transplantation in a Parkinson rat model. Proc Natl Acad Sci U S A. 2002 Feb 19;99(4):2344-9. Epub 2002 Jan 08.

## Claims

1. A method for generating matrix-independent neural stem cells *in vitro*, the method comprising:
isolating cells from tissue of the dental follicle of tooth or wisdom tooth,
culturing the isolated cells in serum-containing medium and isolating sphere-forming cells,
culturing the spheres in a serum-free medium comprising bFGF and EGF and mechanically dissociating the primary spheres,
generating spheroids by culturing the sphere-forming cells again in a serum-free medium comprising bFGF and EGF, and
culturing the spheroids in a serum-containing or serum-free medium comprising bFGF and EGF, wherein undifferentiated neural stem cells are isolated from a cell monolayer in serum-containing medium or from spheroids in serum-free medium.

2. The method of claim 1, wherein the dental follicle stem cells are enzymatically isolated from soft tissue of wisdom teeth.

3. The method of claim 1 or 2, wherein the obtained neural stem cells are further differentiated to neural cells.

4. Use of dental follicle stem cells obtained from tissue of the dental follicle of tooth or wisdom tooth for the production of matrix-independent neural stem cells *in vitro*.

5. Use of dental follicle stem cells obtained from tissue of the dental follicle of tooth or wisdom tooth in the preparation of a medicament for the treatment of neurodegenerative diseases.

6. The use of claim 5, wherein the neurodegenerative disease is selected from the group consisting of Alzheimer's disease, Parkinson's disease, prion diseases, Creutzfeldt-Jakob, Huntington's disease, multiple sclerosis, frontotemporal dementia (Pick's Disease) or amyotrophic lateral sclerosis (ALS or Lou Gehrig's Disease).

## Patentansprüche

1. Verfahren zur Erzeugung matrixunabhängiger neuraler Stammzellen *in vitro*, wobei das Verfahren umfasst:
Isolieren von Zellen aus Gewebe des dentalen Follikels eines Zahns oder Weißheitszahns,
Kultivieren der isolierten Zellen in serumhaltigem Medium und Isolieren von Sphären-bildenden Zellen,
Kultivieren der Sphären in einem serumfreien Medium mit bFGF and EGF und mechanisches Zerteilen der primären Sphären,
Erzeugen von Sphäroiden durch erneutes Kultivieren der Sphären-bildenden Zellen in einem serumfreien Medium mit bFGF and EGF und
Kultivieren der Sphäroiden in einem serumhaltigen oder serumfreien Medium mit bFGF and EGF, wobei undifferenzierte neurale Stammzellen in serumhaltigem Medium aus einer einzelnen Zellschicht oder in einem serumfreien Medium aus Spheroiden isoliert werden.

2. Verfahren nach Anspruch 1, wobei die Stammzellen aus dentalem Follikel enzymatisch aus Weichgewebe von Weißheitszähnen isoliert werden.

3. Verfahren nach Anspruch 1 oder 2, wobei die erzeugten neuralen Stammzellen weiter zu neuralen Zellen differenziert werden.

4. Verwendung der aus Gewebe des dentalen Follikels von Zähnen oder Weißheitszähnen erzeugten Stammzellen des dentalen Follikels zur Produktion von matrixunabhängigen neuralen Stammzellen *in vitro*.

5. Verwendung der aus Gewebe des dentalen Follikels von Zähnen oder Weißheitszähnen erzeugten Stammzellen des dentalen Follikels bei der Herstellung eines Medikaments zur Behandlung von neurodegenerativen Erkrankungen.

6. Verwendung nach Anspruch 5, wobei die neurodegenerative Erkrankung ausgewählt ist aus der Gruppe bestehend aus der Alzheimer-Erkrankung, Parkinson-Krankheit, Prion-Erkrankungen, Creutzfeldt-Jakob-Krankheit, Huntingtons Krankheit, multiple Sklerose, frontotemporaler Demenz (Picks Krankheit) oder amyotrophisch lateraler Sklerose (ALS oder Lou-Gehrigs-Krankheit).

## Revendications

1. Procédé de préparation *in vitro* de cellules souches neurales qui sont indépendantes de la matrice, ledit procédé comprenant :
l'isolation de cellules à partir de tissu du follicule dentaire d'une dent ou d'une dent de sagesse,
la mise en culture des cellules isolées dans un milieu contenant du sérum et l'isolation des cellules formant des sphères,
la mise en culture des sphères dans un milieu exempt de sérum avec du bFGF et du EGF et la division mécanique des sphères primaires,
la préparation de sphéroïdes par la remise en culture des cellules formant des sphères dans un milieu exempt de sérum avec du bFGF et du EGF et
la mise en culture des sphéroïdes dans un milieu contenant du sérum ou un milieu exempt de sérum avec du bFGF et du EGF, les cellules souches étant isolées, dans un milieu contenant du sérum, à partir d'une couche unicellulaire ou bien, dans un milieu exempt de sérum, à partir de sphéroïdes.

2. Procédé selon la revendication 1, les cellules souches issues du follicule dentaires étant isolées de façon enzymatique à partir de tissu mou issu de dents de sagesse.

3. Procédé selon les revendications 1 ou 2, les cellules souches neurales préparées subissant une différenciation ultérieure pour obtenir des cellules neurales.

4. Utilisation des cellules souches du follicule dentaire préparées à partir de tissu du follicule dentaire de dents ou de dents de sagesse pour produire *in vitro* des cellules souches neurales qui sont indépendantes de la matrice.

5. Utilisation des cellules souches du follicule dentaire préparées à partir de tissu du follicule dentaire de dents ou de dents de sagesse lors de la préparation d'un médicament destiné au traitement de maladies neurodégénératives.

6. Utilisation selon la revendication 5, ladite maladie neurodégénérative étant choisie dans le groupe constitué par la maladie d'Alzheimer, la maladie de Parkinson, les affections liées à des prions, la maladie de Creutzfeld-Jakob, la chorée de Huntington, la sclérose en plaques, la maladie de Pick ou la sclérose latérale amyotrophique (SLA ou maladie de Charcot).
